(19) Europäisches Patentamt
European Patent Office
Office européen des brevets

(11) Publication number: **0 264 138 B1**

(12) **EUROPEAN PATENT SPECIFICATION**

(45) Date of publication of patent specification: **04.12.91**

(51) Int. Cl.5: **C07G 11/00**, C12P 1/04, A61K 35/74, C12N 1/20

(21) Application number: **87115147.8**

(22) Date of filing: **16.10.87**

(54) **Novel compound DC-102 and process for production thereof.**

(30) Priority: **16.10.86 JP 246238/86**

(43) Date of publication of application:
**20.04.88 Bulletin 88/16**

(45) Publication of the grant of the patent:
**04.12.91 Bulletin 91/49**

(84) Designated Contracting States:
**DE FR GB**

(56) References cited:

**None**

(73) Proprietor: **KYOWA HAKKO KOGYO CO., LTD.**
**6-1, Ohte-Machi Itchome**
**Chiyoda-ku Tokyo(JP)**

(72) Inventor: **Nakano, Hirofumi**
**1-12-3, Asahi-machi**
**Machida-shi Tokyo(JP)**
Inventor: **Hara, Mitsunobu**
**Popuragaoka Coopu 11-202 2-10-2, Naruse**
**Machida-shi Tokyo(JP)**
Inventor: **Asano, Kozo**
**3-9-10, Naka-Machi**
**Machida-shi Tokyo(JP)**
Inventor: **Yoshida, Mayumi**
**9-18, Isobe**
**Sagamihara-shi Kanagawa-ken(JP)**
Inventor: **Morimoto, Makoto**
**203-5, Shimotogari Nagaizumi-cho**
**Sunto-gun Shizuoka-ken(JP)**

(74) Representative: **Casalonga, Axel et al**
**BUREAU D.A. CASALONGA - JOSSE Morassistrasse 8**
**W-8000 München 5(DE)**

EP 0 264 138 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a novel anti-tumor antibiotic having benzodiazepin nucleus, DC-102, and a process for the production thereof.

Anthramycin, mazethramycin, tomaymycin, neothramycin, sybiromycin, SEN-215, cyclopenia, etc. are known as anti-tumor antibiotics having benzcdiazepin nucleus (CRC Handbook of Antibiotic Compounds, 5, 181, CRC Press, USA, 1981). However, there has been a constant demand for substances having a high anti-tumor activity.

### Brief Description of the Drawings

Figure 1 shows the UV absorption spectra of DC-102 in methanol, in which the solid line represents the data under neutral conditions, the broken line the data under acidic conditions (0.01 N HCl), and the alternate long and short dash line the data under alkaline conditions (0.01 N NaOH).

Figure 2 shows the IR absorption spectrum of DC-102 (KBr disk).

Figure 3 shows $^{13}C$-NMR spectrum of DC-102 (100 MHz, $CD_3OD$, internal standard TMS).

### Detailed Description of the Invention

DC-102 showing high anti-tumor and antibacterial activities has the following physicochemical properties:

(a) Molecular weight and molecular formula

Molecular weight: 461, molecular formula: $C_{24}H_{35}N_3O_6$

It is known that compounds having benzodiazepin nucleus such as tomaymycin have EI mass spectra with no molecular ion peak, in which M+-32 peak corresponding to the molecular species in which MeOH has been released is observed as the maximum ion peak.

The same seems to apply to DC-102.

(b) Mass spectrometry

429.2261 (EI mass spectrometry)

(c) Ultraviolet absorption spectrum (in MeOH)

As shown in Figure 1.

(d) Infrared absorption spectrum (KBr disk)

As shown in Figure 2.

(e) $^{13}C$-NMR spectrum (100 MHz, in $CD_3$ OD internal standard TMS)

As shown in Figure 3.

(f) Elemental analysis

C = 61.47%, H = 7.52%, N = 8.67%

(g) Solubility

Soluble in methanol, acetone and water; insoluble in ethyl acetate, chloroform and hexane.

(h) A white, basic substance.

(i) Thin layer chromatography

Table 1 shows the Rf values when DC-102 is chromatographed on silica gel (Kieselgel 60 Art. 5715; Merck, West Germany) and developed with various solvent systems at room temperature for one hour.

Table 1

| Solvent system | Rf |
|---|---|
| Chloroform : Methanol (5:1) | 0.38 |
| Ethyl acetate : Methanol (3:1) | 0.20 |
| Methanol | 0.51 |

The developed spots of DC-102 can be detected by bioassay using *Bacillus subtilis*, by color development with hot sulfuric acid, ninhydrin reagent or Ehrlich's reagent, or under ultraviolet light.

Of the physicochemical properties shown above, the $^{13}C$-NMR data revealed that DC-102 has, in its structure, benzodiazepin nucleus represented by the following formula:

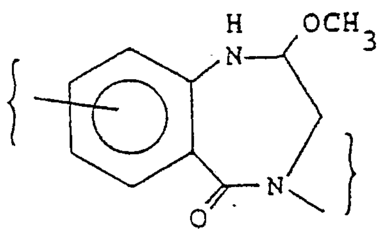

(wherein { and } each represent bonding with undetermined residues). DC-102 differs from known anti-tumor antibiotics having benzodiazepin nucleus in molecular formula and other properties. The methyl group in the benzodiazepin nucleus of DC-102 shown above can be readily replaced with a hydrogen atom or another lower alkyl group by treatment with water or lower alcohols other than methanol, as in the case with known anti-tumor antibiotics having benzodiazepin nucleus.

Pharmacological properties of DC-102 are described below.

(A) Antibacterial activity

The antibacterial activity of DC-102 against various bacteria determined by agar dilution method (pH 7.0) is shown in Table 2.

Table 2

| Bacteria Tested | Minimum Inhibitory Concentration (µg/mℓ) DC-102 |
|---|---|
| Staphylococcus aureus ATCC 6538P | 83 |
| Bacillus subtilis No. 10707 | 42 |
| Klebsiella pneumoniae ATCC 10031 | >100 |
| Salmonella typhosa ATCC 9992 | >100 |
| Escherichia coli ATCC 26 | >100 |

(B) Acute toxicity ($LD_{50}$)

The acute toxicity value ($LD_{50}$) was about 1.5 mg/kg when the compound was intraperitoneally administered to mice.

(C) Therapeutical effect against lymphocytic leukemia P388

Five male $CDF_1$ mice each having a weight of about 22 g were used for each group as test animals, and 1 x $10^6$ cells of lymphocytic leukemia P388 were implanted intraperitoneally in the test animals. 24 hours after implantation, 0.2 mℓ of a phosphate buffer saline (hereinafter referred to as PBS) containing DC-102 was intraperitoneally administered. For comparison, 0.2 mℓ of PBS solution containing mitomycin C was intraperitoneally administered 24 hours after implantation of the tumor cells. The mean survival time (MST) after implantation and increased life span (ILS) represented by T/C (T: MST for test groups; C: MST for control group) are shown in Table 3.

Table 3

| Test Compound | Dose (mg/kg) | MST (days) | ILS (T/C) |
|---|---|---|---|
| DC-102 | 1<br>0.5<br>0.25<br>0.125<br>0 | 14.5<br>16.0<br>13.0<br>12.5<br>10.3 | 140<br>154<br>125<br>120<br>- |
| Mitomycin C | 6 | 17.2 | 166 |

The process for producing DC-102 is described below.

DC-102 can be obtained by culturing a DC-102-producing strain belonging to the genus Streptomyces in a medium and recovering DC-102 formed and accumulated in the culture broth.

Any microorganism can be used in the process of the present invention so long as it is a strain belonging to the genus Streptomyces and having an ability to produce DC-102. Further, some mutant strains derived from such strains by artificial mutation treatment such as ultraviolet irradiation, X-ray irradiation and treatment with various mutagens or by spontaneous mutation have been found to be capable of producing DC-102 and can also be used in the present invention. A typical strain is DO-102 strain.

Microbiological properties of DO-102 strain are as follows. These properties were determined according to the procedures recommended by International Streptomyces Project (ISP) for characterization of species belonging to the genus Streptomyces [E.B. Shirling and D. Gottlieb: Int. J. Syst. Bacteriol., 16, 313-340 (1966)]. The steric configuration of diaminopimelic acid in the whole cell hydrolyzate was determined by the method of B. Becker, et al. [Appl. Microbiol., 12, 421-423 (1964)]. Morphological study was made by using an optical microscope, and a scanning electron microscope was employed for observing the morphology of spore surface. The color indications are given according to the classification in the Color Harmony Manual (Container Corporation of America, 4th edition, 1958).

(1) Morphology

| | |
|---|---|
| Aerial mycelium: | It is branched but not fragmented. |
| Substrate mycelium: | It is branched but not fragmented. |
| Spore : | Spiral chains of more than 10 spores are formed at the end of simply branched aerial mycelium. |
| Surface of spore : | Smooth |
| Shape and size of spore : | Oval (0.5 to 0.6 × 0.7 to 0.9 µm) |
| Motility of spore : | No movement observed |

Formation of sclerotium and sporangium is not observed.

(2) Color

| | |
|---|---|
| Aerial mycelium : | Gray |
| Substrate mycelium : | Brown to deep brown |
| Soluble pigment : | Yellow to brown pigment produced in small amounts |

(3) Steric configuration of diaminopimelic acid : LL-form

(4) Assimilability of carbon sources:

| | |
|---|---|
| Assimilable ; | Cellobiose, fructose, galactose, glucose, glycerol, maltose, methyl glucoside, starch and xylose |
| Nonassimilable ; | Adonitol, arabinose, erythritol, inositol, lactose, mannitol, melibiose, melezitose, raffinose, rhamnose, salicin, sorbitol and sucrose |

(5) Physiological properties

| | |
|---|---|
| Liquefaction of gelatin : | Negative |

| | |
|---|---|
| Hydrolysis of starch : | Positive |
| Coagulation and peptonization of defatted milk : | Both positive |
| Decomposition of cellulose : | Negative |
| Formation of melanin-like pigment : | Negative |
| Growth temperature range : | 10 to 34° C |
| Resistance to NaCl : | Can grow at NaCl concentration up to 5% (w/v). |

Resistance to antibiotics and lysozyme

| | |
|---|---|
| Cephaloridine (100 μg/mℓ) : | Resistant |
| Novobiocin (20 μg/mℓ) : | Sensitive |
| Penicillin G (10 units) : | Resistant |
| Lysozyme (0.005%, w/v) : | Resistant |

Growth temperature range was determined after two days of culturing, the actions upon gelatin, defatted milk and cellulose were observed after one month of culturing at 28° C, and the other observations were made after two weeks of culturing at 28° C.

(6) Cultural characteristics on various agar media

| Medium | Cultural characteristics |
|---|---|
| Sucrose-nitrate agar medium | G : Moderate<br>AM: Poor, pearl (2ba)<br>SM: Pearl pink (3ca)<br>SP: None |
| Glucose-asparagine agar medium | G : Good<br>AM: Abundant, pussy-willow gray (5cb)<br>SM: Orange rust (4pe) to light tan (3gc)<br>SP: Very light brown |
| Glycerol-asparagine agar medium (ISP 5) | G : Good<br>AM: Moderate, bisque (4ec)<br>SM: Oak-brown (4pi)<br>SP: Light mustard tan (2ic) |
| Starch agar medium (ISP 4) | G : Good<br>AM: Abundant, light melon-yellow (3ea) to pussy-willow gray (5cb)<br>SM: Clove-brown (3ni)<br>SP: Very light yellow |
| Tyrosine agar medium (ISP 7) | G : Good<br>AM: Abundant, pussy-willow gray (5cb)<br>SM: Camel (3ie)<br>SP: None |

| Medium | | Cultural characteristics |
|---|---|---|
| Nutrient agar medium | G : | Moderate |
| | AM: | Moderate, nude tan (4gc) |
| | SM: | Oak-brown (4pi) to orange rust (4pe) |
| | SP: | Very light yellow |
| Yeast extract-malt extract agar medium (ISP 2) | G : | Good |
| | AM: | Natural (2dc) to pussy-willow gray (5cb) |
| | SM: | Dark brown (4pn) |
| | SP: | Very light yellow |
| Oatmeal agar medium (ISP 3) | G : | Good |
| | AM: | Abundant, natural (2dc) |
| | SM: | Natural (2dc) |
| | SP: | Very light gray |
| Peptone-yeast extract-iron agar medium (ISP 6) | G : | Good |
| | AM: | Abundant, sand (3cb) |
| | SM: | Camel (3ie) to light melon-yellow (3ea) |
| | SP: | Very light yellow |
| Hickey-Tresner agar medium | G : | Good |
| | AM: | Moderate, sand (3cb) |
| | SM: | Camel (3ie) |
| | SP: | Very light brown |

The above data were obtained after 21 days of culturing at 28° C, in which G represents growth, AM the formation of aerial mycelium and its color, SM the color of substrate mycelium, and SP the formation of soluble pigment and its color.

(7) Identification of DO-102 strain

DO-102 strain belongs to the Type I cell wall group according to the classification of Lechevalier and Lechevalier [M.P. Lechevalier and H.A. Lechevalier: Int. J. Syst. Bacteriol., 20, 435-443, (1970)], since LL-diaminopimelic acid is contained in the whole cell hydrolyzate. On the basis of this characteristic and morphological characteristics, this strain should reasonably be designated as a strain of the genus Streptomyces Waksman and Henrich (1943). Therefore, this strain was designated as a strain of the genus Streptomyces, named Streptomyces sp. DO-102, and deposited with the Fermentation Research Institute, Agency of Industrial Science and Technology under FERM BP-1163 on August 29, 1986.

For the culturing of the microorganism of the present invention, conventional methods for culturing

Actinomycetes are generally used. As the medium, either a natural medium or a synthetic medium can be employed so long as it contains proper amounts of assimilable carbon sources, nitrogen sources, inorganic materials, and substances capable of promoting the growth of the microorganism used and its production of DC-102.

As the carbon source, glucose, starch, dextrin, mannose, fructose, sucrose, lactose, xylose, arabinose, mannitol, molasses, etc. can be used either alone or in combination. Hydrocarbons, alcohols, organic acids, etc. can also be used depending on the assimilation ability of the microorganism. As the nitrogen source, ammonium chloride, ammonium sulfate, ammonium nitrate, sodium nitrate, urea, peptone, meat extract, yeast extract, dry yeast, corn steep liquor, soybean meal, Casamino acid, etc. can be used either alone or in combination. If necessary, inorganic salts such as sodium chloride, potassium chloride, magnesium sulfate, calcium carbonate, potassium dihydrogen phosphate, dipotassium hydrogen phosphate, ferrous sulfate, calcium chloride, manganese sulfate, zinc sulfate and copper sulfate may be added to the medium. In addition, substances capable of promoting the growth of the microorganism used and its production of DC-102 can be employed in trace amounts.

Culturing is usually carried out by liquid culture, and most preferably by submerged culture with stirring. Culturing temperature is 25 to 40°C, preferably 28 to 38°C. It is desirable to maintain the pH of the medium at 4 to 10, preferably 6 to 8, by the addition of aqueous ammonia or aqueous ammonium carbonate during the culturing.

Usually after one to seven days of liquid culture, DC-102, the desired substance, is formed and accumulated in the culture liquor and microbial cells. Culturing is discontinued when the amount of the product in the culture broth reaches the maximum.

Isolation and purification of DC-102 are carried out by the methods usually used for the isolation and purification of microbial metabolic products from culture broth. For example, the culture broth is separated into microbial cells and culture filtrate by filtration, and the separated microbial cells are extracted with a suitable solvent such as chloroform, acetone, etc. The extract is combined with the culture filtrate, and the combined solution is passed through a column packed with a nonionic porous resin such as Diaion HP-20® (Mitsubishi Chemical Industries Ltd.) for absorption of the active component contained. The adsorbed active component is eluted with a suitable solvent such as methanol, acetone, etc. and the eluate is concentrated under reduced pressure, followed by dilution with water. The resulting solution is passed through a column packed with a cation exchange resin such as Diaion SK 104® ($NH_4^+$ type)
(Mitsubishi Chemical Industries Ltd.) for adsorption of the active component, and the adsorbed active component is eluted with a salt-containing aqueous solution. The active component thus eluted is further purified by adsorption on HP-20, column chromatography on silica gel and other means, whereby DC-102 is obtained as white powder.

During the cultivation and purification steps, DC-102 can be traced by bioassay using Bacillus subtilis No. 10707, or by measuring UV absorption of thin layer chromatograms.

DC-102 can be used as an anti-tumor agent in suitable dosage forms prepared by combination with at least one pharmaceutical diluent, adjuvant or carrier. For example, DC-102 is usually dissolved in physiological saline, glucose solution, lactose solution or mannitol solution to prepare injections and administered intravenously to mammals, particularly human beings, in a dose of 0.008-0.88 mg/kg. It may also be administered intra-arterially, intraperitoneally or intrathoracically in similar doses. Freeze-drying according to the method specified in Pharmacopoeia of Japan may be applied to solutions containing DC-102, and injectable powder can be prepared by adding sodium chloride to freeze-dried DC-102. The pharmaceutical preparations of this compound may also contain pharmaceutically acceptable well-known diluents, adjuvants and/or carriers such as pharmaceutically acceptable salts. When DC-102 is used as an injection, it is preferable, in some cases, to use an additive that enhances the solubility of this active ingredient. Doses of DC-102 may be adjusted appropriately depending on the age and conditions of patients. Administration schedule may also be adjusted depending on the dose, as well as the age and conditions of patients; intermittent administration, for example, once a week or once every three weeks, may be adopted. DC-102 may also be orally or rectally administered in similar doses and in the similar manner. For oral or rectal administration, it is used in the form of tablets, powder, granules, syrup or suppository with conventional adjuvants.

The anti-tumor agents thus prepared are expected to be effective against chronic lymphocytic leukemia, chronic myeloid leukemia, breast cancer, stomach cancer, hepatoma, colon cancer, rectal carcinoma, lung cancer, pancreatic cancer, uterine carcinoma, cephalic and cervical tumors, etc. The suitable content of DC-102 in the above anti-tumor agents is 0.01 to 20 mg in 20 to 50 mℓ in the case of injection and 0.001 to 85 weight % when they are used in the form of tablets, capsules, powder, granules and suppositories.

Certain specific embodiments of the present invention are illustrated by the following representative

example and reference example.

Example 1

Streptomyces sp. DO-102 was used as a seed strain. The strain was inoculated into 300 ml of a seed medium in a 2 ℓ-Erlenmeyer flask comprising 5 g/ℓ Bacto-Tryptone (a product of Difco Co., Ltd.), 5 g/ℓ yeast extract, 3 g/ℓ meat extract, 10 g/ℓ soluble starch, 10 g/ℓ glucose and 5 g/ℓ calcium carbonate (pH 7.2, prior to sterilization), and subjected to shaking culture (200 rpm) at 30 °C for 48 hours.

The resulting seed culture (750 mℓ) was inoculated into 15 ℓ of a medium having the same composition as mentioned above in a 30 ℓ-jar fermentor, and cultured at 28 °C for 24 hours (rotation: 200 rpm, aeration: 15 ℓ/min). The resulting culture (15 ℓ) was then transferred to 150 ℓ of a fermentation medium with the following composition in a 200 ℓ-tank fermentor, and cultured with agitation and aeration (200 rpm, 15 ℓ/min) at 28 °C.

Composition of the fermentation medium:
50 g/ℓ dextrin, 10 g/ℓ dry yeast, 10 g/ℓ
$NaNH_4HPO_4 \cdot 4H_2O$, 0.5 g/ℓ $KH_2PO_4$, 0.5 g/ℓ
$MgSO_4 \cdot 7H_2O$, 10 g/ℓ potassium chloride and 5 g/l calcium carbonate (pH 7.2; adjusted with NaOH prior to sterilization)

Culturing was conducted for 70 hours without controlling the pH of the medium. The microbial cells and other precipitates formed were separated from the culture broth by filtration to give 100 ℓ of a filtrate.

The culture filtrate thus obtained was passed through a column packed with 2 ℓ of a polystyrene adsorbent resin Diaion HP-20 for adsorption of the active component contained, and the resin was washed with deionized water to remove impurities, followed by elution with methanol. The active fractions of the eluate were concentrated and the concentrate was diluted with water and passed through a column packed with 2 ℓ of a cation exchange resin SK104 ($NH_4^+$ type) for adsorption of the active component contained. After the resin was washed with deionized water to remove impurities, the adsorbed active component was eluted with a mixed solvent of methanol and 2 M ammonium acetate solution (1:1 by volume). The active fractions of the eluate were concentrated, and the concentrate was again passed through a column packed with HP-20, followed by elution with methanol. The eluate thus obtained was concentrated to dryness, whereby 0.3 g of crude DC-102 was obtained as brown powder. This was applied to a column packed with silica gel (Wakogel C-200; a product of Wako Pure Chemical Industries, Ltd.) , and elution was carried out with chloroform and then with a mixed solvent of chloroform and methanol (7:3 by volume). The fractions containing the active component were concentrated to dryness and the obtained powder was applied to a pressure column packed with silica gel (Lichroprep Si 60; a product of Merck). Elution was carried out with ethyl acetate and then with a mixed solvent of ethyl acetate and methanol (20:1 by volume). After the fraction containing DC-102 was concentrated, n-hexane was added to the concentrate, and the precipitate which separated out was collected and dried under reduced pressure to afford 5 mg of DC-102 as white powder. The physicochemical properties, antibacterial activity and anti-tumor activity of DC-102 thus obtained are as described above.

Reference Example (Injection)

DC-102 (10 mg) was dissolved in 50 mℓ of ethanol, and after stirring, ethanol was removed under reduced pressure. The residue thus obtained was dissolved in about 10 mℓ of sterile physiological saline solution to obtain injections.

**Claims**

**1.** Novel compound DC-102 obtainable by culturing the microorganism Streptomyces sp. DO-102 (FERM BP-1163) and having the following physicochemical properties:
(a) Molecular weight and molecular formula:
Molecular weight; 461.
Molecular formula; $C_{24}H_{35}N_3O_6$.
(b) Mass spectrometry:
429.2261 (EI mass spectrometry)
(c) Ultraviolet absorption spectrum (in MeOH):
As shown in Figure 1

(d) Infrared absorption spectrum (KBr disk):
As shown in Figure 2
(e) $^{13}C$-NMR spectrum:
(100 MHz, $CD_3OD$, internal standard TMS):
As shown in Figure 3
(f) Elemental analysis :
C = 61.47%, H = 7.52%, N = 8.67%
(g) Solubility:
Soluble in methanol, acetone and water; insoluble in ethyl acetate, chloroform and hexane.
(h) A white, basic substance.
(i) Thin layer chromatography:
Table 1 shows the Rf values when DC-102 is chromatographed on silica gel (Kieselgel 60 Art. 5715; Merck, West Germany) and developed with various solvent systems at room temperature for one hour.

Table 1

| Solvent system | Rf |
|---|---|
| Chloroform : Methanol (5:1) | 0.38 |
| Ethyl acetate : Methanol (3:1) | 0.20 |
| Methanol | 0.51 |

2. A process for producing DC-102 which comprises culturing in a medium a microorganism belonging to the genus Streptomyces and capable of producing DC-102, until a substantial amount of DC-102 is accumulated in the culture broth, and recovering DC-102 therefrom.

3. The process according to claim 2, wherein the microorganism is Streptomyces sp. DO-102 (FERM BP-1163).

4. A pharmaceutical composition comprising a pharmaceutical carrier and as an active ingredient, an effective amount of DC-102.

5. A biologically pure culture of Streptomyces sp. DO-102 (FERM BP-1163) which posesses an ability to produce DC-102.

6. Compound DC-102 as defined in Claim 1 to be applied as medicament.

7. Use of the compound or the composition according to Claim 1 and Claim 4, respectively, for the manufacture of a medicament for the application as an anti-tumor or anti-bacterial agent.

**Revendications**

1. Nouveau composé DC-102, que l'on peut obtenir en cultivant le microorganisme Streptomyces sp. DO-102 (FERM BP-1163) et qui présente les propriétés physicochimiques suivantes :
(a) Masse moléculaire et formule moléculaire
Masse moléculaire : 461
Formule moléculaire : $C_{24}H_{35}N_3O_6$
(b) Spectrométrie de masse
429,2261 (spectrométrie de masse EI)
(c) Spectre d'absorption UV (dans MeOH)
représenté sur la Figure 1.
(d) Spectre d'absorption IR (pastille de KBr)
représenté sur la Figure 2.
(e) Spectre de RMN de $^{13}C$ (100 MHz, dans $CD_3OD$, référence interne TMS)
représenté sur la Figure 3.
(f) Analyse élémentaire

C : 61,47 % ; H : 7,52 % ; N : 8,67 %.
(g) Solubilité
soluble dans le méthanol, l'acétone et l'eau ; insoluble dans l'acétate d'éthyle, le chloroforme et l'hexane.
(h) Substance blanche, basique.
(i) Chromatographie sur couche mince :
le Tableau 1 donne les valeurs de $R_f$ obtenues quand on soumet DC-102 à une chromatographie sur gel de silice (Kieselgel 60, Article 5715, Merck, RFA) et que l'on effectue le développement avec divers systèmes solvants, à la température ambiante, pendant une heure.

Tableau 1

| Système solvant | Rf |
|---|---|
| Chloroforme : méthanol (5:1) | 0,38 |
| Acétate d'éthyle : méthanol (3:1) | 0,20 |
| Méthanol | 0,51 |

2. Procédé de production de DC-102, qui comporte la culture, dans un milieu, d'un microorganisme appartenant au genre Streptomyces et capable de produire DC-102, jusqu'à ce qu'une quantité importante de DC-102 se soit accumulée dans le bouillon de culture, et la récupération de DC-102 à partir de celui-ci.

3. Procédé conforme à la revendication 2, dans lequel le microorganisme est Streptomyces sp. DO-102 (FERM BP-1163).

4. Composition pharmaceutique comprenant un véhicule pharmaceutique et, comme ingrédient actif, une quantité efficace de DC-102.

5. Culture biologiquement pure de Streptomyces sp. DO-102 (FERM BP-1163) qui possède la capacité de produire DC-102.

6. Composé DC-102, tel que défini dans la revendication 1, destiné à être utilisé comme médicament.

7. Utilisation du composé conforme à la revendication 1 ou de la composition conforme à la revendication 4, pour la fabrication d'un médicament destiné à être utilisé comme agent antitumoral ou antibactérien.

**Patentansprüche**

1. Durch Anzucht des Mikroorganismus Streptomyces sp. DO-102 (FERM BP-1163) erhältliche neue Verbindung DC-102 mit nachstehenden physikochemischen Eigenschaften:
(a) Molekulargewicht und Molekularformel:
Molekulargewicht; 461
Molekularformel; $C_{24}H_{35}N_3O_6$
(b) Massenspektrometrie:
429,2261 (EI-Massenspektrometrie)
(c) Ultraviolettabsorptionsspektrum (in Methanol):
Wie in Fig. 1 dargestellt
(d) Infrarotabsorptionsspektrum (KBr-Preßling):
wie in Fig. 2 dargestellt
(e) $^{13}$C-NMR-Spektrum:
(100 MHz, $CD_3OD$, innerer Standard TMS):
wie in Fig. 3 dargestellt
(f) Elementaranalyse:
C = 61,47 %, H = 7,52 %, H = 8,67 %
(g) Löslichkeit:
Löslich in Methanol, Aceton und Wasser; unlöslich in Essigsäureethylester, Chlorform und Hexan

(h) Ein weißer, basischer Stoff
(i) Dünnschichtchromatographie:
Tabelle 1 zeigt die Rf-Werte, falls DC-102 an Kieselgel (Kieselgel 60 Art. 5715; Merck, West-Deutschland) chromatographiert und mit verschiedenen Lösungsmittelsystemen bei Raumtemperatur für 1 Stunde entwickelt wird

Tabelle 1

| Lösungsmittelsystem | Rf |
|---|---|
| Chloroform : Methanol (5:1) | 0,38 |
| Essigsäureethylester : Methanol (3:1) | 0,20 |
| Methanol | 0,51 |

**2.** Verfahren zur Herstellung von DC-102, umfassend die Anzucht eines zur Gattung Streptomyces gehörenden und zur DC-102-Erzeugung befähigten Mikroorganismus in einem Medium, bis eine wesentliche Menge an DC-102 in der Nährbrühe angereichert ist und Isolieren von DC-102 daraus.

**3.** Verfahren nach Anspruch 2, wobei der Mikroorganismus Streptomyces sp. DO-102 (FERM BP-1163) ist.

**4.** Arzneimittel, enthaltend einen pharmazeutischen Träger und als einen Wirkstoff eine wirksame Menge von DC-102.

**5.** Biologisch reine Kultur von Streptomyces sp. DO-102 (FERM BP-1163), die die Fähigkeit zur Erzeugung von DC-102 besitzt.

**6.** Verbindung DC-102 gemäß Anspruch 1, angewandt als Medikament.

**7.** Verwendung der Verbindung oder Zusammensetzung nach Anspruch 1 bzw. Anspruch 4 zur Herstellung eines Arzneimittels zur Anwendung als ein anti-Tumor- oder antibakterielles Mittel.

FIG. 1

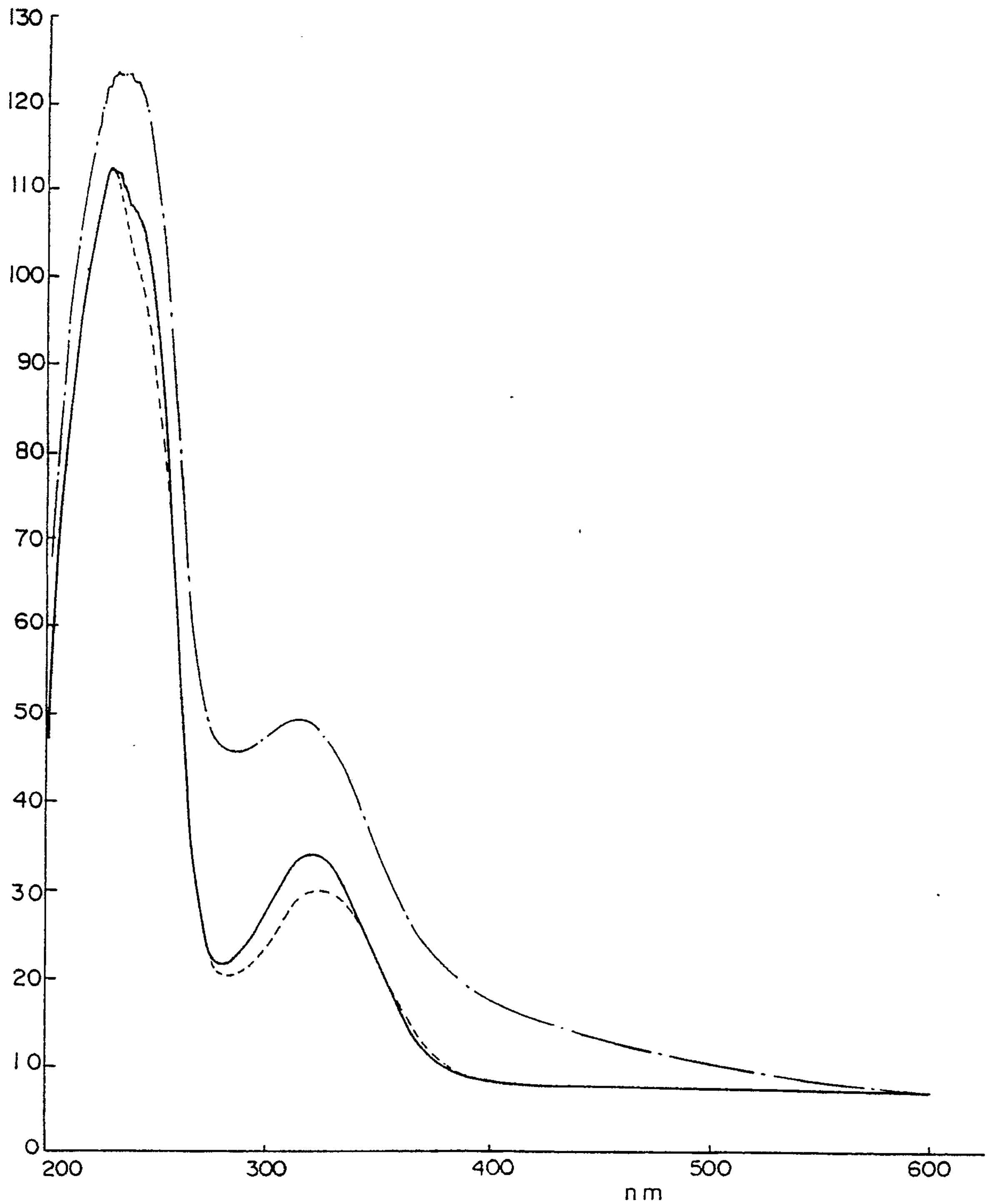

FIG.2

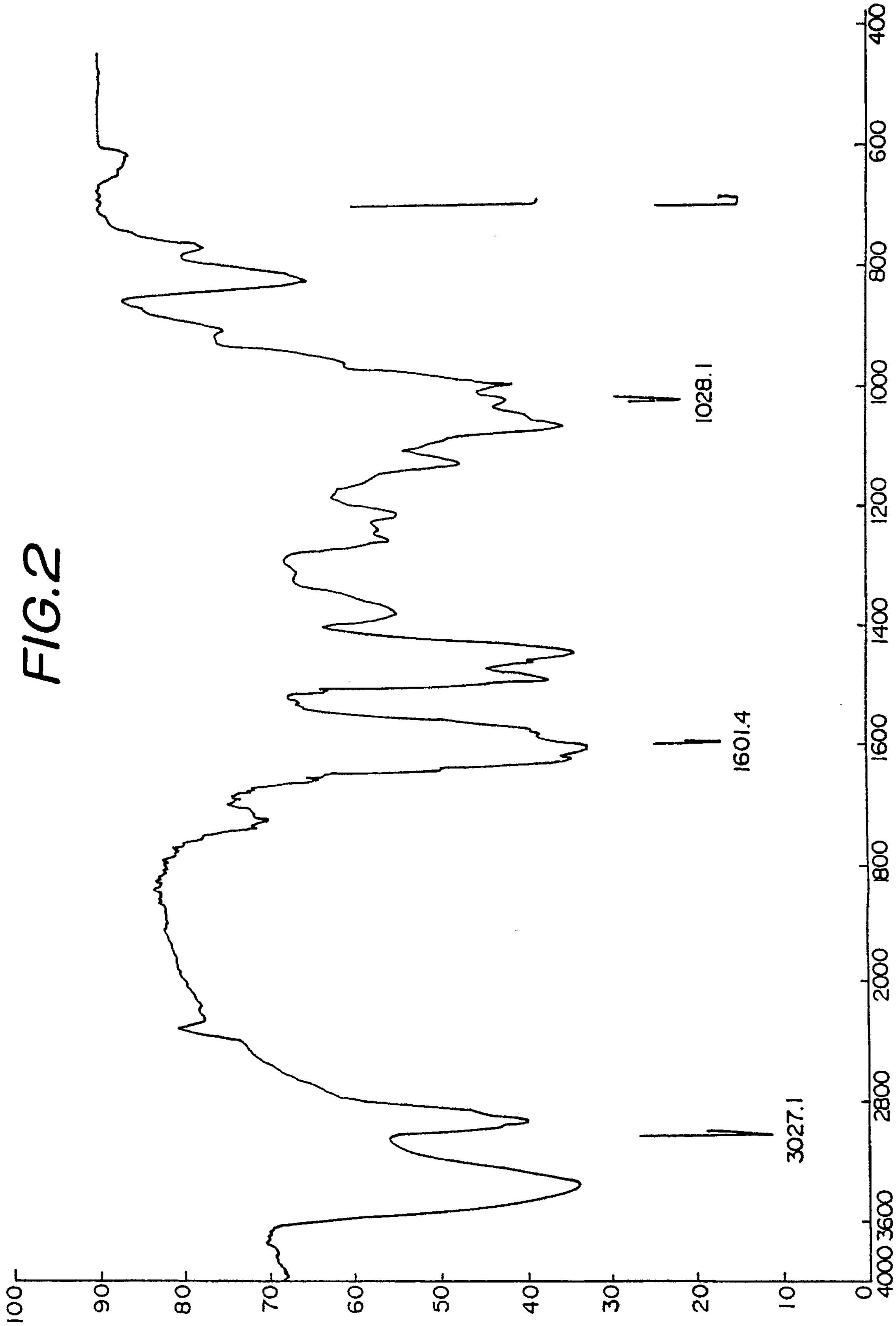
3027.1
1601.4
1028.1
100
90
80
70
60
50
40
30
20
10
0
4000
3600
2800
2000
1800
1600
1400
1200
1000
800
600
400

FIG.3

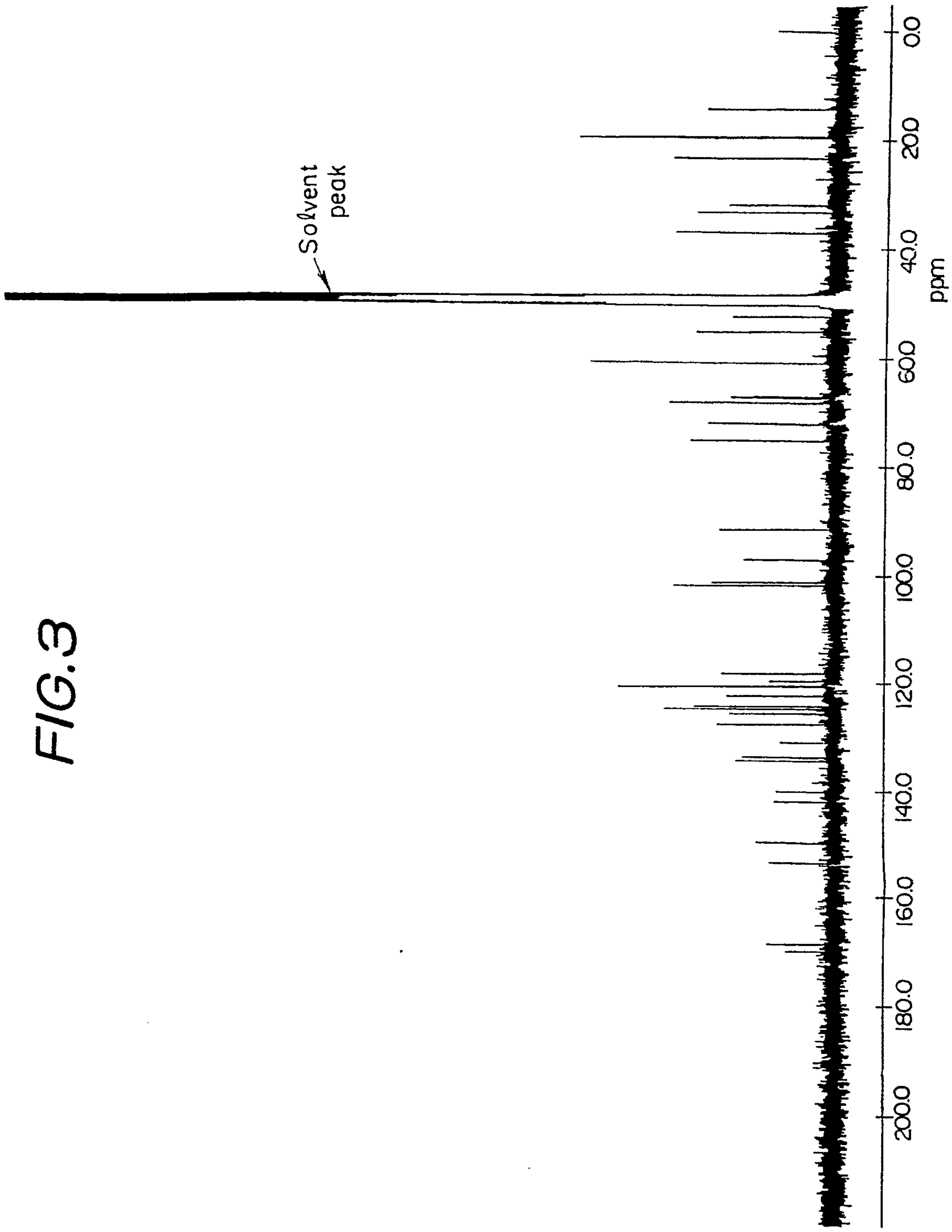
Solvent
peak
0.0
20.0
40.0
60.0
80.0
100.0
120.0
140.0
160.0
180.0
200.0
ppm